(19) Europäisches Patentamt
European Patent Office
Office européen des brevets

(11) **EP 2 103 292 B1**

(12) **EUROPÄISCHE PATENTSCHRIFT**

(45) Veröffentlichungstag und Bekanntmachung des Hinweises auf die Patenterteilung:
**11.05.2016 Patentblatt 2016/19**

(51) Int Cl.:
***A61F 13/44*** *(2006.01)*

(21) Anmeldenummer: **09002983.6**

(22) Anmeldetag: **03.03.2009**

(54) **Textiler oder auf Vliesbasis hergestellter OP-Artikel, insbesondere Kompresse, Tupfer, Bauchtuch**

Textile or non-woven fabric OP article, in particular compress, swab, stomach cloth

Article opératoire textile ou à base de non-tissé, notamment compresse, coton, bandeau ventral

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **11.03.2008 DE 102008014874**

(43) Veröffentlichungstag der Anmeldung:
**23.09.2009 Patentblatt 2009/39**

(73) Patentinhaber: **Paul Hartmann Aktiengesellschaft 89522 Heidenheim (DE)**

(72) Erfinder: **Halbauer, Rainer 73569 Obergröningen (DE)**

(74) Vertreter: **DREISS Patentanwälte PartG mbB Postfach 10 37 62 70032 Stuttgart (DE)**

(56) Entgegenhaltungen:
**GB-A- 716 923      US-A- 2 698 270
US-A- 3 464 415      US-A- 3 941 132
US-A- 5 112 325      US-A1- 2007 219 516**

**Beschreibung**

Die Erfindung betrifft einen auf Vliesbasis hergestellten OP-Artikel, insbesondere zum einmaligen Gebrauch, insbesondere Kompresse, Tupfer, Bauchtuch, mit einem langgestreckten Röntgenkontrastmittel, welches mit einem flächenhaften Träger des OP-Artikels durch ein fadenförmiges Befestigungsmittel verbunden ist.

Derartige OP-Artikel sind vielfach bekannt geworden, s. z. B. US-A-3,464,415 oder US 2007/0219516 A1. Derartige OP-Artikel werden bei Operationen verwendet und in Körperöffnungen während der Operation eingebracht. Hierfür werden diese Artikel häufig aus ihrer herstellerseitig gefalteten Konfiguration gelöst und bauschartig geknüllt. Dabei werden auch Kräfte auf das langgestreckte Röntgenkontrastmittel ausgeübt, und nicht selten besteht das Problem, dass sich das Röntgenkontrastmittel von dem Träger des OP-Artikels ganz oder teilweise löst. Auch die Fixierung des Röntgenkontrastmittels auf dem Träger durch Verschweißen bringt diesbezüglich keine Vorteile. Eine starre Schweißverbindung neigt sogar eher dazu, dass sich das Röntgenkontrastmittel bei Einwirken von Zugspannungen von dem Träger des OP-Artikels löst.

Das Röntgenkontrastmittel dient dem Zweck, dass hierdurch feststellbar ist bzw. sichergestellt werden kann, dass sämtliches OP-Material aus einer Operationswunde, also aus dem Körper des Patienten, entfernt worden ist.

Mit GB 716,923 wurde zur Verbesserung der Auffindbarkeit von chirurgischem Material bereits vorgeschlagen, ein Röntgenkontrastmittel nicht linear sondern bezüglich des Trägers in einer hin- und hergehenden Wellenform zu fixieren, wobei hierfür vorzugsweise eine stoffschlüssige Fixierung eingesetzt werden soll, wobei aber auch eine Nähverbindung alternativ erwähnt ist. US 2007/0219516 A1 offenbart in einem Ausführungsbeispiel die Fixierung eines linear erstreckten Röntgenkontrastmittels mittels eines Nähfadens. US 5,112,325 lehrt ein sehr spezielles Röntgenkontrastmittel aus einem Bündel von einzelnen Polymerfäden mit röntgenkontrastfähigem Material, welches durch Hitze und/oder Druck fixiert werden soll. US 3,464,415 offenbart die Fixierung eines linear erstreckten Röntgenkontrastmittels mittels eines Paars von Nähfäden, wobei anschließend durch Einleitung von Hitze und Druck das Röntgenkontrastmittel erweicht wird, so dass die Materialien miteinander innig verbunden werden.

Der vorliegenden Erfindung liegt die Aufgabe zugrunde, OP-Artikel der vorstehend genannten Art noch weiter dahingehend zu verbessern, dass die Handhabung und die Betriebssicherheit dieser Artikel verbessert wird.

Diese Aufgabe wird durch einen OP-Artikel mit den Merkmalen des Anspruchs 1 gelöst.

Mit der Erfindung wurde erkannt, dass das typischerweise faden-, schnur- oder bandförmige Röntgenkontrastmittel, wenn es wellenförmig oder mäandrierend auf den Träger aufgebracht wird, wesentliche Vorteile mit sich bringt. Zum einen behindert das Röntgenkontrastmittel eine Dehnung des OP-Artikels in geringerem Maße, weil die Wellen- oder Mäanderform gewissermaßen ein ausdehnbares Reservoir darstellt. Auf diese Weise kann ein Röntgenkontrastmittel verwendet werden, dessen Dehnbarkeit auch deutlich geringer sein kann als diejenige des Trägers des OP-Artikels. Es erweist sich hier auch im besonderen Maße als vorteilhaft, dass das Röntgenkontrastmittel durch ein fadenförmiges Befestigungsmittel mit dem Träger verbunden ist und nicht durch eine starre Schweißverbindung. Auf diese Weise ist das wellenförmig oder mäandrierend aufgebrachte Röntgenkontrastmittel insbesondere schwimmend, jedoch gleichwohl verliersicher an dem Träger gehalten. Bei Dehnung des Trägers in Längsrichtung des Röntgenkontrastmittels kann dieses von der Wellen- oder Mäanderform in eine Linienform übergehen, ohne dass das Röntgenkontrastmittel einer tatsächlichen Dehnung oder Streckung seines Materials ausgesetzt wäre, welche bei der Handhabung des OP-Artikels als unangenehm oder störend empfunden würde oder zu einem Loslösen des Röntgenkontrastmittels von dem Träger führen würde.

Außerdem erweist es sich als vorteilhaft, dass das fadenförmige Befestigungsmittel einen ersten Nähfaden und einen zweiten Nähfaden umfasst, die miteinander und mit dem Röntgenkontrastmittel zusammenwirken, und dass der eine Nähfaden das langgestreckte Röntgenkontrastmittel umgreift und der andere Nähfaden den Träger des OP-Artikels untergreift. Solchenfalls bilden die Nähfäden beidseits neben dem Röntgenkontrastmittel einen Schlingungspunkt oder Kreuzungspunkt, mittels dessen die Nähfäden unter Zwischenordnung des Röntgenkontrastmittels gegeneinander und gegen den Träger festziehbar sind.

Weiter wird erfindungsgemäß vorgeschlagen, dass auch das fadenförmige Befestigungsmittel für das Röntgenkontrastmittel wellenförmig oder mäandrierend verläuft, also nicht lediglich im Wesentlichen eng anliegend an das Röntgenkontrastmittel entlang dessen Längserstreckung verläuft, sondern selbst eine Wellen- oder Mäanderform bildet. Solchenfalls erweist es sich als vorteilhaft, wenn das fadenförmige Befestigungsmittel um 180° versetzt zu der Wellen- oder Mäanderform des langgestreckten Röntgenkontrastmittels verläuft und dieselbe Wellen- oder Periodenlänge wie dieses aufweist. Solchenfalls ist die Wellen- oder Mäanderform des fadenförmigen Befestigungsmittels zu derjenigen des Röntgenkontrastmittels verschoben.

Die Wellen- oder Periodenlänge ist definiert als der Abstand zwischen zwei gleich gerichteten, periodisch aufeinanderfolgenden Scheitelpunkten der Wellen- oder Mäanderform.

Die Wellen- oder Mäanderform des langgestreckten Röntgenkontrastmittels weist eine Höhe auf. Die Höhe der Wellen- oder Mäanderform ist dabei definiert als die doppelte Amplitude der Wellen- oder Mäanderform. Gemessen wird die Amplitude zwischen den jeweils äußersten Kanten des langgestreckten Röntgenkontrastmittels bzw. des fadenförmigen Befestigungsmittels.

In besonders vorteilhafter Weise entspricht die Höhe der Wellen- oder Mäanderform des langgestreckten Röntgenkontrastmittels im Wesentlichen der Höhe der Wellen- oder Mäanderform des fadenförmigen Befestigungsmittels, wobei dies nicht zwingend erforderlich ist. Es können durchaus diesbezüglich Unterschiede bestehen.

Es hat sich gezeigt, dass die Wellen- oder Periodenlänge des langgestreckten Röntgenkontrastmittels und/oder des fadenförmigen Befestigungsmittels 4 - 15 mm, insbesondere 4 - 12 mm, insbesondere 4 - 10 mm, insbesondere 5 - 9 mm und weiter insbesondere 6 - 9 mm beträgt.

Des Weiteren erweist es sich als vorteilhaft, wenn die Höhe der Wellen- oder Mäanderform des langgestreckten Röntgenkontrastmittels und/oder des fadenförmigen Befestigungsmittels 0,5 - 5 mm, insbesondere 0,5 - 4 mm, insbesondere 0,5 - 3 mm, insbesondere 0,7 - 3 mm und weiter insbesondere 0,7 - 2,5 mm beträgt.

Im Sinne der vorliegenden Erfindung bestehen für das langgestreckte Röntgenkontrastmittel keine zwingenden Vorgaben. Das langgestreckte Röntgenkontrastmittel kann insbesondere fadenförmig, bandförmig, als Einzelfilament oder als Bündel aus Einzelfilamenten gestaltet sein. Das Röntgenkontrastmittel enthält vorzugsweise Bariumsulfat, vorzugsweise mindestens 60 Gew-% Bariumsulfat bezogen auf das Gesamtgewicht des Röntgenkontrastmittels. Das Grundmaterial des langgestreckten Röntgenkontrastmittels umfasst vorzugsweise polymere Materialien aus der Gruppe Polyester, Polyethylen, Polypropylen und/oder Polyvinylchlorid. Insbesondere vorzugsweise umfasst das langgestreckte Röntgenkontrastmittel Polypropylen mit mindestens 60 Gew-% Bariumsulfat.

Jedoch erweist sich die Erfindung als besonders vorteilhaft, wenn das langgestreckte Röntgenkontrastmittel ein Bündel aus Einzelfäden umfasst, die von einer Umhüllung umgeben sind, da derartige Röntgenkontrastmittel typischerweise wenig dehnbar sind und ihre Verwendung deshalb häufig zu den eingangs geschilderten Problemen führt. Die genannte Umhüllung derartiger Röntgenkontrastmittel ist typischerweise gesponnen, was die Dehnbarkeit weiter beeinträchtigt.

OP-Artikel sind insbesondere Kompressen, Bauchtücher oder Tupfer, insbesondere zum einmaligen Gebrauch.

Der Träger des OP-Artikels kann vorzugsweise ein Vlies umfassen.

Der Träger des OP-Artikels kann insbesondere ein Vlies aus Viskose und Polyester umfassen, insbesondere mit 60 bis 80 Gew.-% Viskose und 40 bis 20 Gew.-% Polyester. Es sei an dieser Stelle auch darauf hingewiesen, dass als Träger auch Verbundmaterialien, insbesondere Verbundmaterialien aus Vliesstoffen und/oder textilen Materialien (wie insbesondere Gewirke, Gewebe, Gestricke) Verwendung finden können.

Der Träger des OP-Artikels kann vorzugsweise ein Vlies mit Verfestigung durch Wasserstrahlvernadelung (Spunlaced Vlies) umfassen.

Insbesondere vorzugsweise kann der Träger des OP-Artikels ein Spunlaced Vlies mit 60 bis 80 Gew.-% Viskose und 40 bis 20 Gew.-% Polyester umfassen.

Das Vlies hat vorzugsweise ein Flächengewicht von 20 g/m² bis 90 g/m², weiter vorzugsweise ein Flächengewicht von 30 g/m² bis 80 g/m².

Weiter vorzugsweise umfasst der Träger für eine Kompresse ein Vlies mit einem Flächengewicht von 50 g/m² bis 90 g/m², insbesondere von 60 g/m² bis 80 g/m².

Weiter vorzugsweise umfasst der Träger für eine Kompresse ein Spunlaced Vlies mit 60 bis 80 Gew.-% Viskose und 40 bis 20 Gew.-% Polyester und mit einem Flächengewicht von 50 g/m² bis 90 g/m², insbesondere von 60 g/m² bis 80 g/m².

Weiter vorzugsweise umfasst der Träger für einen Tupfer ein Vlies mit einem Flächengewicht von 20 g/m² bis 60 g/m², insbesondere von 30 g/m² bis 50 g/m².

Weiter vorzugsweise umfasst der Träger für einen Tupfer ein Spunlaced Vlies mit 60 bis 80 Gew.-% Viskose und 40 bis 20 Gew.-% Polyester und mit einem Flächengewicht von 20 g/m² bis 60 g/m², insbesondere von 30 g/m² bis 50 g/m².

Vorzugsweise wird das langgestreckte Röntgenkontrastmittel in Maschinenrichtung des Trägers aufgebracht.

In Weiterbildung der Erfindung weist der Träger insbesondere vorteilhafte Bruchdehnungen auf.

Die Bruchdehnung des Trägers wird als die am Messpunkt der Bruchkraft maximal erreichte Dehnung (ausgedrückt in %) verstanden.

Die Bruchkraft beschreibt die maximale Kraft (ausgedrückt in N) im Kräfteverlauf der Messung, bei der der definierte Probenkörper vollständig zerreißt und damit zerstört wird.

Aus dem Träger werden verteilt jeweils 5 Probenkörper in Maschinenrichtung des Trägers (MD) und in Querrichtung zur Maschinenrichtung (CD) ausgestanzt.

Als Prüfkörper werden einlagige Stanzlinge mit einer Dimension von 50 mm Breite x 260 mm Länge aus dem Träger ausgestanzt.

Alternativ können auch Prüfkörper mit geringeren Dimensionen vermessen werden, wenn Träger mit nur geringerer Längenerstreckung zur Verfügung stehen. Entsprechend werden kürzere Einspannlängen von 100 mm, 50 mm oder 25 mm eingestellt.

Der Prüfkörper wird spannungslos über mindestens 24 h bei 23°C und 50% relativer Luftfeuchtigkeit klimatisiert.

Die Prüfkörper werden trocken oder nass wie nachfolgend beschrieben geprüft: Für die Nassprüfung werden die Prüflinge bis zur vollständigen Benetzung in 0,05%iger Triton X-100-Lösung (Triton X-100: Merck Artikel-Nr. 12298; in demineralisiertem Wasser) gelegt, danach entnommen, überschüssige Lösung abgestreift und dann unverzüglich wie folgt geprüft. Der Prüfkörper wird in eine vertikale Zugprüfeinrichtung nach EN ISO 75001 eingespannt, wobei der Abstand für die Einspannvorrichtung, also die Ausgangslänge L0 200 mm beträgt. Hierbei wird der Prüfkörper über seine gesamte Breite (50 mm) an einem Ende in die feststehende Klemmbacke (mit einer Klemmenbreite von 50 mm) fest eingespannt. An dem anderen Ende wird der Abschnitt über seine gesamte Breite in die bewegliche Klemmbacke der Zugprüfeinrichtung fest eingespannt und es wird eine Vorkraft von 0,1 N angelegt. Die bewegliche Klemmbacke wird mit einer Geschwindigkeit von 100 mm/min bis zum vollständigen Bruch (vollständiges Zerreißen) des Prüfkörpers bewegt. Es wird eine Kraft-Dehnungskurve aufgezeichnet. Anschließend wird die gedehnte Länge L1 des Abschnittes bei Bruch des Probenkörpers in mm gemessen.

Die Bruchdehnung in % berechnet sich dann nach folgender Formel:

$$Dehnung[\%] = \frac{L1[mm] - L0[mm]}{L0[mm]} x100\%$$

Es werden jeweils 5 Probenkörper vermessen.

Besonders bevorzugt ist der Träger des OP-Artikels ein Vlies mit einer Bruchdehnung im trockenen Zustand in Maschinenrichtung (MD) von durchschnittlich 35 - 70 %, weiter vorzugsweise von 40 - 65%, weiter vorzugsweise von 45 - 60 %.

Besonders bevorzugt ist der Träger des OP-Artikels ein Vlies mit einer Bruchdehnung im trockenen Zustand quer zur Maschinenrichtung (also in Cross Direction CD) von durchschnittlich 50 - 75 %, weiter vorzugsweise von 55 - 70 %.

Besonders bevorzugt ist der Träger des OP-Artikels ein Vlies mit einer Bruchdehnung im nassen Zustand in Maschinenrichtung (MD)von durchschnittlich 45 - 70 %, weiter vorzugsweise von 50 - 65 %.

Besonders bevorzugt ist der Träger des OP-Artikels ein Vlies mit einer Bruchdehnung im nassen Zustand entgegen der Maschinenrichtung (also in Cross Direction CD) von durchschnittlich 50 - 75 %, weiter vorzugsweise von 50 - 70 %, weiter vorzugsweise von 50 - 65 %.

Vorzugsweise ist die Kraft K1, die der Träger einer Verformung im elastischen Bereich entgegensetzt, geringer als die Kraft K2, die das Röntgenkontrastmittel einer Verformung im elastischen Bereich entgegensetzt. Der E-Modul des Trägermaterials ist also vorzugsweise geringer als der des Röntgenkontrastmittels.

Weiter sei erwähnt, dass das langgestreckte Röntgenkontrastmittel typischerweise auf eine eine Oberfläche des OP-Artikels bildende Sichtseite des Trägers aufgebracht ist. Es wäre indessen aber auch denkbar, dass das Röntgenkontrastmittel zwischen Flachmaterialien, also innerhalb eines sandwichartigen Verbunds, angeordnet ist. Es ist auch denkbar, dass der aus Flachmaterialien hergestellte Träger des OP-Artikels herstellerseitig gefaltet vorliegt.

Weitere Einzelheiten, Merkmale und Vorteile ergeben sich aus den beigefügten Patentansprüchen und aus der zeichnerischen Darstellung und nachfolgenden Beschreibung einer bevorzugten Ausführungsform des erfindungsgemäßen OP-Artikels. In der Zeichnung zeigt:

Figur 1 eine Draufsicht auf einen ausgestanzten Abschnitt eines erfindungsgemäßen OP-Artikels in Form einer Vliesstoffkompresse mit einem langgestreckten Röntgenkontrastmittel;

Figur 2 eine Seitenansicht zur Verdeutlichung der Anfügung des Röntgenkontrastmittels an einen Träger des OP-Artikels nach Figur 1 und

Figur 3 eine Draufsicht gesehen in Richtung des Pfeils III in Figur 2.

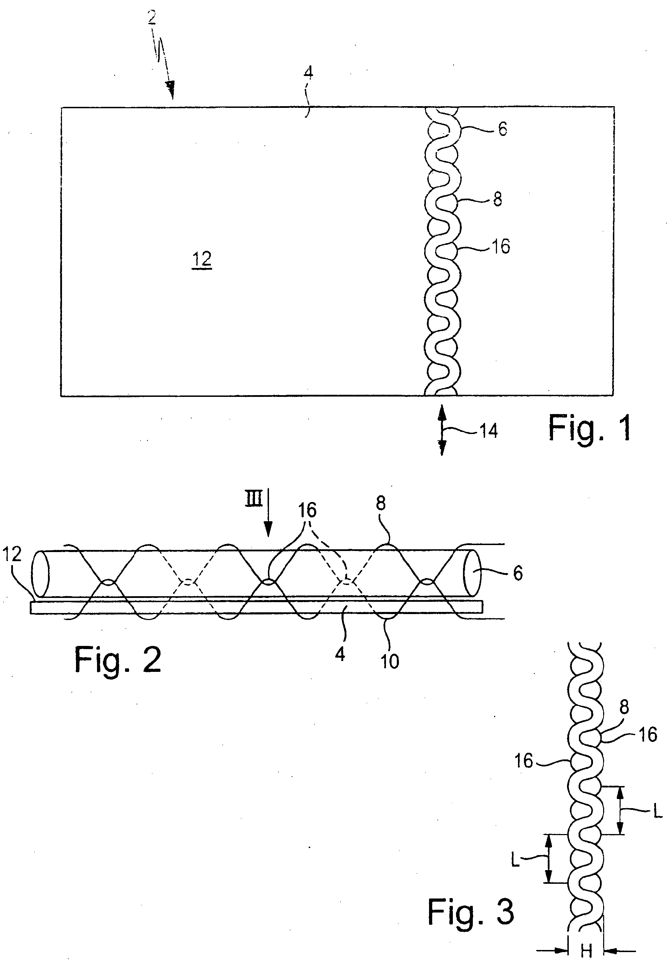

Figur 1 zeigt einen Abschnitt eines flächenhaft erstreckten OP-Artikels in Form einer Vliesstoffkompresse oder eines sonstigen auf Vliesbasis hergestellten Flachmaterials zur Verwendung im OP-Bereich, welcher z. B. in eine Tupferform bringbar, also unregelmäßig faltbar ist, oder in eine Kompressenform bringbar, also regelmäßig faltbar ist. Der OP-Artikel umfasst eine oder mehrere Schichten, die einen flächenhaften Träger 4 bilden, sowie ein langgestrecktes Röntgenkontrastmittel 6, welches mittels noch näher zu beschreibender Nähfäden 8, 10 (s. Figur 2) an dem Träger 4 verliersicher, jedoch nicht 100%-ig stoffschlüssig, sondern vorzugsweise in einem geringen Maß verschieblich fixiert ist.

Man erkennt aus den Figuren, dass das Röntgenkontrastmittel 6 wellenförmig oder mäandrierend auf der Oberfläche 12 des Trägers 4 angeordnet und in dieser Wellen- oder Mäanderform mittels der Nähfäden 8, 10 fixiert ist. Hierdurch wird erfindungsgemäß erreicht, dass bei einer Dehnung des OP-Artikels 2, insbesondere in Richtung des Doppelpfeils 14, eine Längung des Röntgenkontrastmittels 6 möglich ist, indem die Wellen- oder Mäanderform in eine ungefähre Linienform übergeht, und zwar ohne dass in das Material des Röntgenkontrastmittels 6 streckende oder dehnende Kräfte eingeleitet würden. Hierdurch wird einerseits erreicht, dass das Röntgenkontrastmittel 6 die Handhabung, insbesondere Dehnung des OP-Artikels bei seiner Verwendung nicht behindert, und andererseits wird verhindert, dass das Röntgenkontrastmittel 6 bei einer Dehnung des OP-Artikels beschädigt wird. Des Weiteren erweist es sich als besonders vorteilhaft, dass das Röntgenkontrastmittel 6 mittels Nähfäden 8, 10 (und nicht durch Schweißung) gegen den Träger 4 befestigt ist, da die beschriebene Längung des OP-Artikels 2 ohne weiteres möglich ist, und zwar ohne dass sich das Röntgenkontrastmittel 6 von dem Träger 4 löst.

Eine hierfür bevorzugte Führung der Nähfäden 8, 10 ist aus den Figuren 2 und 3 ersichtlich. Dabei ist der eine Nähfaden 8 ausschließlich auf der einen Seite des Trägers 4 vorgesehen und umschlingt dabei das Röntgenkontrastmittel 6. Der Nähfaden 8 bildet dabei selbst eine Wellen- oder Mäanderform, welche hinsichtlich ihrer Wellen- oder Periodenlänge L derjenigen des Röntgenkontrastmittels 6 entspricht. Diese Wellen- oder Periodenlänge L ist im beispielhaft dargestellten Fall vorzugsweise 6 bis 9 mm. Die Höhe H der Wellen- oder Mäanderform des Röntgenkontrastmittels 6 beträgt typischerweise 0,5 bis 5 mm. Sie entspricht in vorteilhafter Weise im Wesentlichen der Höhe der Wellen- oder Mäanderform der Nähfäden 8, 10, wobei dies nicht zwingend erforderlich ist. Es können durchaus diesbezüglich Unterschiede bestehen.

Der zweite Nähfaden 10 erstreckt sich im Wesentlichen auf der von dem Röntgenkontrastmittel 6 abgewandten Seite des Trägers 4. In Figuren 1 und 3 sind lediglich Kreuzungspunkte 16 auf der dem Röntgenkontrastmittel zugewandten Oberseite 12 des Trägers 4 ersichtlich. Der jeweilige Kreuzungspunkt 16 wurde also durch Festziehen des Nähfadens 10 gegen die Oberfläche 12 des Trägers 4 gezogen.

Je größer die Höhe der Wellen- oder Mäanderform des oder der Nähfäden 8, 10, desto größer ist die Beweglichkeit des Röntgenkontrastmittels 6 gegenüber der Oberfläche 12 des Trägers.

### Patentansprüche

1. Auf Vliesbasis hergestellter OP-Artikel (2), insbesondere zum einmaligen Gebrauch, insbesondere Kompresse, Tupfer, Bauchtuch, mit einem langgestreckten Röntgenkontrastmittel (6), welches mit einem flächenhaften Träger (4) des OP-Artikels durch ein fadenförmiges Befestigungsmittel (8, 10) verbunden ist, **dadurch gekennzeichnet, dass** das Röntgenkontrastmittel (6) in der Draufsicht auf die Oberfläche (12) des flächenhaften Trägers (4) wellenförmig oder mäandrierend aufgebracht ist und dass das fadenförmige Befestigungsmittel (8, 10) einen ersten Nähfaden (8) und einen zweiten Nähfaden (10) umfasst, die miteinander zusammenwirken, und dass der eine Nähfaden (8) das langgestreckte Röntgenkontrastmittel (6) umgreift und der andere Nähfaden (10) den Träger (4) untergreift und dass das fadenförmige Befestigungsmittel (8, 10) ebenfalls wellenförmig oder mäandrierend verläuft und um 180° versetzt zu der Wellen- oder Mäanderform des langgestreckten Röntgenkontrastmittels (6) verläuft und dieselbe Wellen- oder Periodenlänge (L) wie dieses aufweist.

2. OP-Artikel nach einem oder mehreren der vorstehenden Ansprüche, **dadurch gekennzeichnet, dass** die Wellen- oder Periodenlänge (L) des langgestreckten Röntgenkontrastmittels (6) und/oder des fadenförmigen Befestigungsmittels (8, 10) 4 - 15 mm, insbesondere 4 - 12 mm, insbesondere 4 - 10 mm, insbesondere 5 - 9 mm und weiter insbesondere 6 - 9 mm beträgt.

3. OP-Artikel nach einem oder mehreren der vorstehenden Ansprüche, **dadurch gekennzeichnet, dass** die Höhe (H) der Wellen- oder Mäanderform des langgestreckten Röntgenkontrastmittels (6) und/oder des fadenförmigen Befestigungsmittels (8, 10) 0,5 - 5 mm, insbesondere 0,5 - 4 mm, insbesondere 0,5 - 3 mm, insbesondere 0,7 - 3 mm und weiter insbesondere 0,7 - 2,5 mm beträgt.

4. OP-Artikel nach einem oder mehreren der vorstehenden Ansprüche, **dadurch gekennzeichnet, dass** das langgestreckte Röntgenkontrastmittel (6) ein Bündel aus Einzelfäden umfasst, die von einer Umhüllung umgeben sind.

5. OP-Artikel nach Anspruch 4, **dadurch gekennzeichnet, dass** die Umhüllung gesponnen ist.

6. OP-Artikel nach einem oder mehreren der vorstehenden Ansprüche, **dadurch gekennzeichnet, dass** der Träger (4) ein Vlies aus Viskose und Polyester umfasst.

7. OP-Artikel nach Anspruch 6, **dadurch gekennzeichnet, dass** der Träger (4) ein Vlies aus 60 - 80 Gew.-% Viskose und 40 - 20 Gew.-% Polyester umfasst.

### Claims

1. An OR article (2) produced from nonwoven fabric, in particular to be used only once, in particular compresses, swabs, abdominal bandage, having an elongated radiological contrast agent (6), which is connected to a two-dimensional substrate (4) of the OR article by a filamentlike fastening means (8, 10), **characterized in that** the radiological contrast agent (6), in plan view, is applied in wavelike or meandering fashion to the surface (12) of the two-dimensional substrate (4), and that the filamentlike fastening means (8, 10) includes a first sewing thread (8) and a second sewing thread (10) which cooperate with one another, and that the one sewing thread (8) wraps around the elongated radiological contrast agent (6), and the other sewing thread (10) engages the substrate (4) from beneath, and that the filamentlike fastening means (8, 10) likewise extends in wavelike or meandering fashion and extends offset by 180° from the wavelike or meandering form of the elongated radiological contrast agent (6) and has the same wave length or period length (L) as the radiological contrast agent.

**2.** The OR article of one or more of the foregoing claims, **characterized in that** that the wave length or period length (L) of the elongated radiological contrast agent (6) and/or of the filamentlike fastening means (8, 10) is from 4-15 mm, in particular 4-12 mm, in particular 4-10 mm, in particular 5-9 mm, and further in particular 6-9 mm.

**3.** The OR article of one or more of the foregoing claims, **characterized in that** the height (H) of the wavelike or meandering form of the elongated radiological contrast agent (6) and/or of the filamentlike fastening means (8, 10) amounts to 0.5-5 mm, in particular 0.5-4 mm, in particular 0.5-3 mm, in particular 0.7-3 mm, and further in particular 0.7-2.5 mm.

**4.** The OR article of one or more of the foregoing claims, **characterized in that** the elongated radiological contrast agent (6) includes a bundle of individual threads that are surrounded by a sheath.

**5.** The OR article of claim 4, **characterized in that** the sheath is spun.

**6.** The OR article of one or more of the foregoing claims, **characterized in that** the substrate (4) includes a nonwoven of viscose and polyester.

**7.** The OR article of claim 6, **characterized in that** the substrate (4) includes a nonwoven comprising from 60-80 weight-% viscose and from 40-20 weight-% polyester.

## Revendications

**1.** Article opératoire (2) à base d'un non-tissé, destiné en particulier à un usage unique, en particulier compresse, tampon, bandeau ventral, comprenant un moyen de contraste radiographique allongé (6), lequel est relié à un support plan (4) de l'article opératoire par un moyen de fixation (8, 10) en forme de fil, **caractérisé en ce que** le moyen de contraste radiographique (6), en vue en élévation de la surface (12) du support plan (4), est appliqué de façon sinueuse et **en ce que** le moyen de fixation (8, 10) en forme de fil comprend un premier fil de couture (8) et un deuxième fil de couture (10), qui coopèrent l'un avec l'autre, et **en ce que** l'un des fils de couture (8) vient se plaquer autour du moyen de contraste radiographique allongé (6) et l'autre fil de couture (10) vient se plaquer sous le support (4), et **en ce que** le moyen de fixation (8, 10) en forme de fil s'étend également de façon sinueuse et est décalé de 180° par rapport à la forme sinueuse du moyen de contraste radiographique allongé (6) et présente la même longueur d'onde ou de période (L) que ce dernier.

**2.** Article opératoire selon l'une ou plusieurs des revendications précédentes, **caractérisé en ce que** la longueur d'onde ou de période (L) du moyen de contraste radiographique (6) allongé et/ou du moyen de fixation (8, 10) en forme de fil atteint 4-15 mm, en particulier 4-12 mm, en particulier 4-10 mm, en particulier 5-9 mm, et plus particulièrement 6-9 mm.

**3.** Article opératoire selon l'une ou plusieurs des revendications précédentes, **caractérisé en ce que** la hauteur (H) de la forme sinueuse du moyen de contraste radiographique allongé (6) et/ou du moyen de fixation (8, 10) en forme de fil atteint 0,5-5 mm, en particulier 0,5-4 mm, en particulier 0,5-3 mm, en particulier 0,7-3 mm et plus particulièrement 0,7-2,5 mm.

**4.** Article opératoire selon l'une ou plusieurs des revendications précédentes, **caractérisé en ce que** le moyen de contraste radiographique allongé (6) comprend un paquet de fils individuels qui sont entourés par une enveloppe.

**5.** Article opératoire selon la revendication 4, **caractérisé en ce que** l'enveloppe est tissée.

**6.** Article opératoire selon l'une ou plusieurs des revendications précédentes, **caractérisé en ce que** le support (4) comprend un non-tissé fait de viscose et de polyester.

**7.** Article opératoire selon la revendication 6, **caractérisé en ce que** le support (4) comprend un non-tissé fait de 60-80 % de viscose et de 40-20 % de polyester.

Fig. 1

Fig. 2

Fig. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 3464415 A
- US 20070219516 A1
- GB 716923 A
- US 5112325 A